**Europäisches Patentamt**

**European Patent Office**

**Office européen des brevets**

(19)

(11) Veröffentlichungsnummer: **0 131 826**
**B1**

# (12) EUROPÄISCHE PATENTSCHRIFT

(45) Veröffentlichungstag der Patentschrift:
**19.11.87**

(21) Anmeldenummer: **84107598.9**

(22) Anmeldetag: **30.06.84**

(51) Int. Cl.⁴: **G 01 N 33/96**

(54) **Lagerstabiles Universalkontrollserum.**

(30) Priorität: **08.07.83 DE 3324626**

(43) Veröffentlichungstag der Anmeldung:
**23.01.85 Patentblatt 85/4**

(45) Bekanntmachung des Hinweises auf die Patenterteilung:
**19.11.87 Patentblatt 87/47**

(84) Benannte Vertragsstaaten:
**AT BE CH DE FR GB IT LI LU NL SE**

(56) Entgegenhaltungen:
**EP - A - 0 005 243**
**EP - A - 0 019 939**
**EP - A - 0 082 587**
**DE - A - 2 617 742**
**FR - A - 2 095 921**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH, Sandhofer Strasse 116, D-6800 Mannheim 31 (DE)**

(72) Erfinder: **Rehner, Helmut, Dr. phil. nat., Am Betberg 39, D-8120 Weilheim (DE)**
Erfinder: **Bartl, Knut, Dr. rer. nat., Am Westend 6, D-8121 Wilzhofen (DE)**
Erfinder: **Portenhauser, Rudolf, Dr. rer. nat., Kustermannstrasse 26 b, D-8132 Tutzing (DE)**

**Beschreibung**

Gegenstand der Erfindung ist ein lagerstabiles Universalkontrollserum auf Basis eines menschlichen oder tierischen Serums, das durch Zusatz eines geeigneten Puffers, dessen Pufferwirkung im physiologischen Bereich liegt, stabilisiert ist.

Zur Richtigkeits- und Präzisionskontrolle von Bestimmungsmethoden bzw. zum Eichen von Analysenautomaten werden üblicherweise Kontrollseren bzw. Eichseren eingesetzt, welche die zu bestimmenden Parameter in bekannten, genau festgelegten Konzentrationen enthalten. Man unterscheidet Spezialkontrollseren, die zur Kontrolle bzw. zur Eichung von ganz bestimmten Parametern oder einer begrenzten, meist zusammengehörigen Gruppe von Parametern (z.B. Gerinnungsparameter) dienen, und sogenannte Universalkontrollseren, die zur Kontrolle bzw. zum Eichen möglichst aller gängigen Parameter nach sämtlichen, in der Praxis üblicherweise durchgeführten Bestimmungsmethoden einsetzbar sind. Sämtliche Substrate und Enzyme, die für die Diagnostik von Bedeutung sind, sind in den derzeit handelsüblichen Universalkontrollseren enthalten. Es lassen sich damit 150 bis 200 Bestimmungsmethoden kontrollieren bzw. eichen.

Die Zuverlässigkeit der Kontrollseren und damit die Zuverlässigkeit der mit den Kontrollseren kontrollierten bzw. geeichten Bestimmungsmethoden hängt entscheidend von der Stabilität der Substrate und Enzyme im Kontrollserum ab. Das Stabilitätsproblem ist umso wichtiger, aber auch umso schwieriger zu lösen, je mehr Parameter in dem Kontrollserum enthalten sind. Kontrollseren kommen üblicherweise in lyophilisierter Form in den Handel, da hierbei die Stabilitätsprobleme am geringsten sind. Doch auch die Lyophilisation, die Lagerung des lyophilisierten Produktes und die Rekonstitution des Kontrollserums vor dessen Gebrauch bedingen einen Stabilitätsverlust, der bei einzelnen Parametern beträchtlich sein kann und bei häufig bestimmten, für die Diagnostik besonders wichtigen Paramtern, wie Bilirubin, Creatinkinase, Aspartat-Aminotransferase, Alanin-Aminotransferase, Aldolase u.ä. zu Problemen führt.

Entscheidend bei einem Kontrollserum ist die Wiederfindung der einzelnen Parameter nach Lagerung und Rekonstitution des lyophilisierten Serums. Diese Wiederfindungsrate sollte im Idealfalle bei 100% liegen. Handelsübliche Universalkontrollseren zeigen Wiederfindungsraten, die für die meisten Parameter in dem Bereich zwischen 85 und 95% liegen.

Aufgabe der vorliegenden Erfindung war es, die Stabilität des in die Universalkontrollseren auf Basis eines menschlichen oder tierischen Serums enthaltenen Parameter zu verbessern, so dass die Wiederfindungsraten, insbesondere für in der Praxis häufig gemessene kritische Parameter nahezu bei 100% liegen.

Es wurde überraschenderweise festgestellt, dass die Wiederfindungsrate für die einzelnen Parameter deutlich gesteigert wird, wenn das Universalkontrollserum auf Basis eines menschlichen oder tierischen Serums einen geeigneten Puffer enthält, dessen Pufferwirkung im physiologischen Bereich liegt. Die Steigerung der Wiederfindungsrate liegt erfindungsgemäss für viele Parameter bei 5 bis 10%, so dass in vielen Fällen nahezu der Idealwert 100% erreicht wird.

Es ist bekannt, Spezialkontrollseren zur Erhöhung der Stabilität der darin enthaltenen Substrate bzw. Enzyme einen Puffer zuzusetzen. So ist beispielsweise in Amer. J. Clin. Path. 53 (1970) S. 924 bis 927 eine Kontrollösung für Gerinnungsparameter beschrieben, die durch Zusatz von HEPES-Puffer (HEPES = N-2-Hydroxyethylpiperazino-N'-2-ethansulfonsäure) stabilisiert worden ist. Andere Puffer enthaltende Spezialkontrollplasmen sind in EP-A-0005243 und in DE-A-2617742 beschrieben. Universalkontrollseren auf Basis eines menschlichen oder tierischen Serums hat man bisher jedoch noch keinen Puffer zugesetzt. Solche Kontrollseren sind seit vielen Jahren in ungepuffertem Zustand im Handel. Bei den zahlreichen Substraten und Enzymen, die in universal einsetzbaren Kontrollseren enthalten sind, hat man bisher den Zusatz von Puffern vermieden, weil zu befürchten war, dass jeder Zusatz einer weiteren chemischen Substanz zu Störungen einzelner Methoden führt.

Darüber hinaus besitzen die Bestandteile von menschlichen und tierischen Seren an sich bereits eine Pufferwirkung. Es wurde bisher für ausreichend erachtet, diese Seren mit dem natürlichen Puffersystem in die lyophilisierte Form überzuführen. Äusserstenfalls hielt man es für notwendig, den nach der Rekonstitution des lyophilisierten Serums beobachteten pH-Wert-Anstieg, der auf einen $CO_2$-Verlust bei der Lyophilisation zurückzuführen ist, auf einen physiologischen pH-Wert zu korrigieren, um zu verhindern, dass bestimmte Parameter bei einem zu hohen pH-Wert im rekonstituierten Kontrollserum instabil werden.

Als Puffersubstanzen können prinzipiell alle Puffer eingesetzt werden, deren $p_K$-Werte den physiologischen pH-Bereich umfassen. Besonders geeignet sind Puffer, die sich aus einem Base- (Kation) und einem Säureteil (Anion) zusammensetzen, wobei als Base 2-Amino-2-(hydroxymethyl)-1,3-propandiol, 2-Amino-2-methyl-1,3-propandiol, Imidazol, Diethyl-, Triethyl-, Diethanol-, Triethanol-amin und als Säure Acetat, Propionat, Malat, Maleat, Succinat, Tartrat, Citrat, Borat bevorzugt sind. Ganz besonders vorteilhaft werden zwitterionische Puffer eingesetzt, beispielsweise HEPES (N-2-hydroxyethylpiperazin-N'-2-ethansulfonsäure), PIPES (Piperazin-N-N'-bis-(2-ethansulfonsäure) und MOPS (3-(N-Morpholino)-propansulfonsäure).

Zur Herstellung des erfindungsgemässen lagerstabilen Universalkontrollserums, wird das menschliche oder tierische Serum, beispielsweise Rinderserum, mit den gewünschten Substraten und Enzymen versetzt. Es wird dann die Puffersubstanz zugegeben. Die erhaltene Lösung wird filtriert und kann in üblicher Weise lyophilisiert werden. Das Universalkontrollserum wird vorzugsweise in lyophilisierter Form in den Handel gebracht. Das Lyophilisilat kann mit wässrigem Medium, vorzugsweise destilliertem Wasser, rekonstituiert werden.

Der Puffer wird dem Serum vor der Lyophilisation in einer Konzentration von 10 bis 100 mmol/l, vorzugsweise von 30 bis 50 mmol/l zugesetzt. Der pH-Wert des eingesetzten Puffers soll im Bereich 6,0 bis 8,0, vorzugsweise im Bereich 6,7 bis 7,3 liegen.

Das so hergestellte Universalkontrollserum ist besonders stabil. Die Stabilität des Universalkontrollserums kann beispielsweise dadurch bestimmt werden, dass man es einer Temperaturbelastung (z.B. 3 Wochen bei 35 °C) unterwirft und danach die Wiederfindungsrate der einzelnen Parameter bestimmt. Das erfindungsgemässe Universalkontrollserum weist Wiederfindungsraten auf, die für viele Parameter bei nahezu 100% liegen. Es ist daher hinsichtlich der Stabilität den bisher bekannten handelsüblichen ungepufferten Universalkontrollseren deutlich überlegen.

Die Erfindung wird durch die folgenden Beispiele näher erläutert:

Beispiel 1 (Vergleichsbeispiel)
Universalkontrollserum auf Humanserum-Basis ohne Pufferzusatz

100 ml Humanserum werden mit den Enzymen Aldolase, α-Amylase, alkalische Phosphatase (AP), Cholinesterase (CHE), Creatinkinase (CK), Glutamat-dehydrogenase (GIDH), Aspartat-Aminotransferase (GOT), Alanin-Aminotransferase (GPT), L-γ-Glutamyl-Transferase (γ-GT), Leucin-Arylamidase (LAP), Lactat-Dehy-drogenase (LDH), Lipase und saure Phosphatase (SP) in einer Menge versetzt, dass die in Tabelle 1 angegebenen Endkonzentrationen im Serum erreicht werden. Weiterhin werden Bilirubin, Creatinin, Glukose, Harnsäure, Harnstoff, Lactat, Salicylat, Eisen- und Kupfersalze zugesetzt, ebenfalls in einer solchen Menge, dass die in Tabelle 1 angegebenen Endkonzentrationen erreicht werden. Es wird Essigsäure zugegeben. Die Menge wird so bemessen, dass im rekonstituierten Lyophilisat ein pH-Wert von ca. 7,0 erreicht wird. Die erhaltene Lösung wird klar filtriert, in Portionen von 5 ml in Flaschen abgefüllt und lyophilisiert. Die lyophilisierten Proben werden bei Kühlschranktemperatur gelagert.

Ein Teil der Proben wird für 3 Wochen bei 35 °C gehalten. Danach werden zur Überprüfung der Stabilität die Aktivitäten der Enzyme und die Konzentrationen der Substrate in temperaturbelasteten und unbelasteten Proben nach Rekonstitution mit Wasser bestimmt. Die Wiederfindung in der belasteten Probe, verglichen mit der unbelasteten Probe in % sind in der anschliessenden Tabelle 2 aufgeführt.

Beispiel 2
Universalkontrollserum auf Humanserum-Basis mit Pufferzusatz

Zu 100 ml Humanserum werden die in Beispiel 1 angegebenen Enzyme und Substrate zugegeben. Es wird HEPES-Puffer zugesetzt, so dass die Lösung einen pH-Wert von ca. 7,0 aufweist (ca.

1,2 g). Es wird wie in Beispiel 1 angegeben, weiterbearbeitet. Die Wiederfindungsdaten sind ebenfalls in der anschliessenden Tabelle 2 aufgeführt.

Beispiel 3
Universalkontrollserum auf Humanserum-Basis mit Pufferzusatz

100 ml Humanserum wird mit den in Beispiel 1 angegebenen Enzymen und Substraten versetzt. Zusätzlich werden Triglyceride und Cholesterin zugegeben, so dass der in Tabelle 1 aufgeführte Gesamt-Triglycerid- bzw. Gesamt-Cholesterin-Gehalt erreicht wird. Ferner wird HEPES-Puffer zugesetzt, so dass die Lösung einen pH-Wert von ca. 7,0 aufweist (ca. 1,2 g). Die so erhaltene Lösung wird, wie in Beispiel 1 beschrieben, weiterbearbeitet. Die Wiederfindungsdaten sind ebenfalls in der anschliessenden Tabelle 2 aufgeführt.

Beispiel 4
Universalkontrollserum/Universalkalibrator auf Rinderserum-Basis mit Pufferzusatz

100 ml hämolysearmes Rinderserum wird mit den in Beispiel 1 angegebenen Enzymen versetzt. Weiterhin werden Bilirubin, Creatinin, Glukose, Glycerin, Harnsäure, Harnstoff und gegebenenfalls Triglyceride, Cholesterin, Lactat, Salicylat, Eisen-, Kupfer- und Magnesiumsalze zugesetzt, bis die in Tabelle 1 angegebenen Endkonzentrationen erreicht werden. Es wird Triethanolaminsuccinat zugegeben, so dass die Lösung einen pH-Wert von ca. 7,0 aufweist (ca. 2,08 g).

Die so erhaltene Lösung wird klar filtriert und, wie in Beispiel 1 angegeben, weiterbearbeitet. Die Wiederfindungsraten sind ebenfalls in der anschliessenden Tabelle 2 aufgeführt.

In der folgenden Tabelle 1 sind die Endkonzentrationen der verschiedenen Parameter in den Universalkontrollseren nach Beispiel 1 bis 4 angegeben. Die Normal-Werte gelten für ein Universalkontrollserum zur Kontrolle bzw. zum Eichen von Messungen, bei denen Parameter in üblicher, normalerweise auftretenden Konzentrationen bestimmt werden. Die pathologischen Werte gelten für ein Universalkontrollserum zur Kontrolle bzw. zum Eichen von Messungen, bei denen Parameter im pathologischen Konzentrationsbereich bestimmt werden. Bei den Aktivitätsangaben für die eingesetzten Enzyme ist jeweils die Temperatur angegeben, bei der die Aktivität bestimmt wird.

In Tabelle 2 sind die Wiederfindungsraten für die Universalkontrollseren nach Beispiel 1 bis 4 aufgeführt. Die Werte stellen Mittelwerte aus mehrfachen Messungen dar. Die Werte gelten sowohl für Universalkontrollseren, für den Normal-Bereich als auch für den pathologischen Bereich.

Tabelle 1
Endkonzentrationen der verschiedenen Parameter in den Universalkontrollseren nach Beispiel 1 bis 4

| Enzym/Substrat | Normal-Werte | erhöhte (pathologische) Werte |
|---|---|---|
| Aldolase | 14 U/l (37 °C) | 22 U/l (37 °C) |
| α-Amylase | 320 U/l (37 °C) | 600 U/l (37 °C) |
| AP | 280 U/l (22 °C) | 380 U/l (22 °C) |
| CHE | 1500 U/l (25 °C) | 8000 U/l (25 °C) |
| CK | 120 U/l (25 °C) | 230 U/l (25 °C) |
| GlDH | 15 U/l (25 °C) | 20 U/l (25 °C) |
| GOT | 35 U/l (25 °C) | 100 U/l (25 °C) |
| GPT | 35 U/l (25 °C) | 100 U/l (25 °C) |
| γ-GT | 50 U/l (25 °C) | 140 U/l (25 °C) |
| LAP | 50 U/l (25 °C) | 130 U/l (25 °C) |
| LDH | 240 U/l (25 °C) | 380 U/l (25 °C) |
| Lipase | 350 U/l (25 °C) | 55 U/l (25 °C) |
| SP | 21 U/l (37 °C) | 28 U/l (37 °C) |
| Bilirubin | 1,5 mg/dl | 5,0 mg/dl |
| Creatinin | 2,0 mg/dl | 4,0 mg/dl |
| Glukose | 130 mg/dl | 270 mg/dl |
| Harnsäure | 5,0 mg/dl | 10,0 mg/dl |
| Harnstoff | 55 mg/dl | 100 |
| Lactat | 6,0 mg/dl | 20,0 mg/dl |
| Salicylat | 2,0 mg/dl | – |
| Eisen-II-Ionen | 100 µg/dl | – |
| Kupfer-II-Ionen | 150 µg/dl | – |
| Gesamt-Triglyceride | 90 mg/dl | 150 mg/dl |
| Gesamt-Cholestern | 90 mg/dl | 150 mg/dl |
| Mg-Ionen | 2,2 mg/dl | 4,5 mg/dl |

Tabelle 2
Wiederfindung in % nach Belastung des Lyophilisates 3 Wochen bei 35 °C bezogen auf unbelastete Probe

| Enzym/ Substrat | Beisp. 1 Universalkontrollserum Humanserum ohne Puffer | Beisp. 2 Universalkontrollserum Humanserum mit Puffer | Beisp. 3 Universalkontrollserum Humanserum mit Puffer | Beisp. 4 Universalkontrollserum Rinderserum mit Puffer |
|---|---|---|---|---|
| Aldolase | 90 | 97 | 90 | 88 |
| α-Amylase | 91 | 98 | 96 | 95 |
| AP | 93 | 99 | 97 | 97 |
| CHE | 98 | 99 | 99 | – |
| CK | 86 | 96 | 91 | 90 |
| GlDH | 66 | 80 | – | 73 |
| GOT | 90 | 92 | 91 | 95 |
| GPT | 85 | 94 | 93 | 94 |
| γ-GT | 97 | 98 | 102 | 99 |
| LAP | 88 | 96 | 91 | 93 |
| LDH | 93 | 97 | 103 | 97 |
| Lipase | 98 | 99 | 99 | 101 |
| SP | 96 | 98 | 93 | 96 |
| Bilirubin | 93 | 99 | 99 | 98 |
| Glukose | 88 | 94 | 86 | 92 |

**Patentansprüche**

1. Lagerstabiles Universalkontrollserum auf Basis eines menschlichen oder tierischen Serums, dadurch gekennzeichnet, dass es einen ihm zugesetzten Puffer enthält, dessen Pufferwirkung im physiologischen pH-Bereich liegt.

2. Universalkontrollserum gemäss Anspruch 1, dadurch gekennzeichnet, dass als Puffer ein aus einem Base- und einem Säureteil zusammengesetzter Puffer, wobei die Base ausgewählt wird aus 2-Amino-2-(hydroxymethyl)-1,3-propandiol, 2-Amino-2-methyl-1,3-propandiol, Imidazol, Diethyl-, Triethyl-, Diethanol- und Triethanolamin und die Säure ausgewählt wird aus Acetat, Propionat, Malat, Maleat, Succinat, Tartrat, Citrat und Borat, oder ein zwitterionischer Puffer eingesetzt wird.

3. Universalkontrollserum gemäss Anspruch 2, dadurch gekennzeichnet, dass als Puffer Triethanolamin-succinat, N-2-hydroxyethylpiperazin-N'-2-ethansulfonsäure, Piperazin-N,N'-bis(2-ethansulfonsäure) oder 3-(N-morpholino)-propansulfonsäure verwendet wird.

4. Universalkontrollserum gemäss einem der Ansprüche 1 bis 3, dadurch gekennzeichnet, dass der Puffer dem Serum vor der Lyophilisation in einer Konzentration von 10 bis 100 mmol/l zugesetzt wird.

5. Universalkontrollserum gemäss Anspruch 4, dadurch gekennzeichnet, dass die Pufferkonzentration 30 bis 50 mmol/l beträgt.

6. Universalkontrollserum gemäss einem der Ansprüche 1 bis 5, dadurch gekennzeichnet, dass der Puffer eine Pufferwirkung in dem pH-Bereich 6,0 bis 8,0 aufweist.

7. Universalkontrollserum gemäss Anspruch 6, dadurch gekennzeichnet, dass die Pufferwirkung des Puffers in dem Bereich 6,7 bis 7,3 liegt.

8. Universalkontrollserum gemäss einem der Ansprüche 1 bis 7 in lyophilisierter oder mit wässrigem Medium rekonstituierter Form.

**Claims**

1. Storage-stable universal control serum based on a human or animal serum, characterised in that it contains a buffer added to it, the buffer action of which lies in the physiological pH range.

2. Universal control serum according to claim 1, characterised in that, as buffer, there is added a buffer composed of an acid and a base component, whereby the base is selected from 2-amino-2-(hydroxymethyl)-propane-1,3-diol, 2-amino-2-methyl-propane-1,3-diol, imidazole, diethyl-, triethyl-, diethanol- and triethanolamine and the acid is selected from acetate, propionate, malate, maleate, succinate, tartrate, citrate and borate, or a zwitterionic buffer is used.

3. Universal control serum according to claim 2, characterised in that, as buffer, there is used triethanolamine succinate, N-2-hydroxyethylpiperazine-N'-2-ethanesulphonic acid, piperazine-N,N'-bis-(2-ethanesulphonic acid) or 3-(N-morpholino)-propanesulphonic acid.

4. Universal control serum according to one of claims 1 to 3, characterised in that the buffer is added to the serum before lyophilisation in a concentration of 10 to 100 mmol/l.

5. Universal control serum according to claim 4, characterised in that the buffer concentration amounts to 30 to 50 mmol/l.

6. Universal control serum according to one of claims 1 to 5, characterised in that the buffer has a buffer action in the pH range of 6.0 to 8.0.

7. Universal control serum according to claim 6, characterised in that the buffer action of the buffer lies in the pH range of 6.7 to 7.3.

8. Universal control serum according to one of claims 1 to 7 in lyophilised form or in a form reconstituted with an aqueous medium.

**Revendications**

1. Sérum témoin universel stable au stockage, à base d'un sérum humain ou animal, caractérisé par le fait qu'il contient un tampon qui lui a été ajouté et dont l'action de tampon se situe dans la gamme physiologique de pH.

2. Sérum témoin universel selon la revendication 1, caractérisé par le fait que l'on utilise comme tampon un tampon composé d'un fragment basique et d'un fragment acide, la base étant sélectionnée entre le 2-amino-2-(hydroxyméthyl)- 1,3-propanediol, le 2-amino-2-méthyl-1,3-propanediol, l'imidazole, la diéthyl-, triéthyl-, diéthanol- et triéthanolamine, et que l'acide est sélectionné entre l'acétate, le propionate, le malate, la maléate, le succinate, le tartrate, le citrate et le borate, ou que l'on utilise un tampon amphotère.

3. Sérum témoin universel selon la revendication 2, caractérisé en ce que l'on utilise comme tampon le succinate de triéthanolamine, l'acide, N-2-hydroxyéthylpipérazine-N'-2-éthanesulfonique, l'acide pipérazine N,N'-bis-(2-éthanesulfonique) ou l'acide 3-(N-morpholino)-propanesulfonique.

4. Sérum témoin universel selon l'une des revendications 1 à 3, caractérisé par le fait que l'on ajoute le tampon au sérum avant la lyophilisation, à une concentration de 10 à 100 mmol/l.

5. Sérum témoin universel selon la revendication 4, caractérisé en ce que la concentration de tampon est de 30 à 50 mmol/l.

6. Sérum témoin universel selon l'une des revendications 1 à 5, caractérisé en ce que le tampon présente une action de tampon dans la gamme de pH de 6,0 à 8,0.

7. Sérum témoin universel selon la revendication 6, caractérisé en ce que l'action de tampon du tampon se situe dans la gamme de 6,7 à 7,3.

8. Sérum témoin universel selon l'une des revendications 1 à 7, sous forme lyophilisée ou reconstituée avec du milieu aqueux.